# EUROPEAN PATENT APPLICATION

(11) **EP 4 624 897 A1**
(43) Date of publication of application: **01.10.2025**
(21) Application number: 25164272.4
(22) Date of filing: 18.03.2025
(51) Int. Cl.: G01N 17/00, G01W 1/18, B05B 1/02, B05B 1/30, B05B 1/34, B05B 12/16, B05B 16/60, G01N 33/00

(54) **RAINFALL TEST APPARATUS AND RAINFALL TEST METHOD**

(30) Priority: 27.03.2024 JP 2024051109
(71) Applicant: Espec Corp., Kita-ku Osaka-shi Osaka 530-8550 (JP)
(72) Inventor: ENOKI, Hiroyuki, Osaka, 530-8550 (JP); SETO, Haruki, Osaka, 530-8550 (JP)
(74) Representative: SSM Sandmair

(57) **Abstract**

A rainfall test apparatus (10) is a test apparatus that produces rain with a rainfall intensity of 10 mm/h or less to evaluate characteristics of a test specimen against rainfall. The test apparatus includes a plurality of nozzles (20, 20a, 20b), each configured to spray water droplets (25). Each of the plurality of nozzles (20, 20a, 20b) has a configuration in which the spreading range of the sprayed water droplets (25) changes according to supply water pressure. The nozzles (20, 20a, 20b) are installed to spray the water droplets (25) toward above the test specimen such that the water droplets (25) reach the test specimen with a flow direction of the water droplets (25) changing downward from a spraying direction.

## Description

### FIELD OF INVENTION

The present invention relates to a rainfall test apparatus and a rainfall test method.

### BACKGROUND ART

Conventionally, as disclosed in Japanese Unexamined Utility Model Application Publication No. S50-90693, rainfall test apparatuses are known that artificially produce rain on a test specimen to evaluate characteristics of the test specimen against rainfall. The test apparatus disclosed in Japanese Unexamined Utility Model Application Publication No. S50-90693 has a configuration in which a large number of pipes are arranged on a ceiling and a large number of sprinkling nozzles are arranged below each pipe. The sprinkling nozzles drop water supplied through the pipes. This reproduces artificial rainfall.

Since the rainfall test apparatus disclosed in Japanese Unexamined Utility Model Application Publication No. S50-90693 only drops water from the sprinkling nozzles, when trying to increase the rainfall intensity (mm/h), the water flowing out from the sprinkling nozzles will not form water droplets but will become a continuous stream. Meanwhile, by reducing the amount of water flowing out from the sprinkling nozzles, it may be possible to cause raindrop-like water droplets to flow out from the sprinkling nozzles. However, the configuration of dropping water droplets from the large number of sprinkling nozzles arranged along the pipes is not suitable for performing tests with low rainfall intensity of, for example, 10 mm/h or less.

To perform tests with low rainfall intensity, it is necessary to have a configuration in which water droplets are sprayed from a smaller number of sprinkling nozzles and to reduce the amount of water sprayed from each sprinkling nozzle. To reduce the amount of water sprayed from each sprinkling nozzle, the supply water pressure to the sprinkling nozzle needs to be reduced. Therefore, a decrease in the water pressure applied to the sprinkling nozzle can result in a narrower range of spread of water droplets when sprayed from the sprinkling nozzle, which may create areas where no rain falls. Therefore, to reproduce conditions close to actual rainfall, it is necessary to increase the number of sprinkling nozzles, but increasing the number of sprinkling nozzles will increase the total amount of water. Therefore, simply reducing the supply water pressure has limitations in reducing the rainfall intensity. In addition, increasing the number of sprinkling nozzles will also increase the apparatus cost.

### SUMMARY OF THE INVENTION

An object of the present invention is to provide a rainfall test apparatus and a rainfall test method that can perform tests with low rainfall intensity at 10 mm/h or less.

A rainfall test apparatus according to one aspect of the present invention is a test apparatus for evaluating characteristics of a test specimen against rainfall by producing rain with rainfall intensity of 10 mm/h or less, and includes nozzles each configured to spray water droplets. Each of the nozzles has a configuration in which a spreading range of the sprayed water droplets changes according to supply water pressure. At least one of the nozzles is installed to spray the water droplets toward above the test specimen such that the water droplets reach the test specimen with a flow direction of the water droplets changing downward from a spraying direction.

A rainfall test method according to another aspect of the present invention is a test method for evaluating characteristics of a test specimen against rainfall by producing rain with rainfall intensity of 10 mm/h or less, and including spraying water droplets from nozzles having a configuration in which a spreading range of the sprayed water droplets changes according to supply water pressure. At this time, at least one of the nozzles sprays the water droplets toward above the test specimen such that the water droplets reach the test specimen with a flow direction of the water droplets changing downward from a spraying direction.

### BRIEF DESCRIPTION OF THE DRAWINGS

Fig. 1 is a schematic diagram showing a rainfall test apparatus according to a first embodiment;
Fig. 2 is a diagram showing a test area as viewed from above, showing the range where water droplets fall on a floor surface;
Fig. 3 is a diagram for describing that the position where the water droplets fall varies depending on the size of the water droplets;
Fig. 4 is a diagram showing a case where a first nozzle and a second nozzle are disposed at positions facing each other;
Fig. 5 is a diagram showing a case where the nozzles are installed such that spray ports face obliquely upward;
Fig. 6 is a diagram for describing the configuration of the nozzle;
Fig. 7 is a diagram showing one example of a case where the nozzles are installed by changing the position and direction of the nozzles;
Fig. 8 is a schematic diagram showing a rainfall test apparatus according to a second embodiment; and
Fig. 9 is a diagram for describing positions of openings when an outside area exists only on one pair of opposing side surfaces of a test area.

### DETAILED DESCRIPTION

Embodiments for carrying out the present invention will be described in detail below with reference to the drawings.

### (First embodiment)

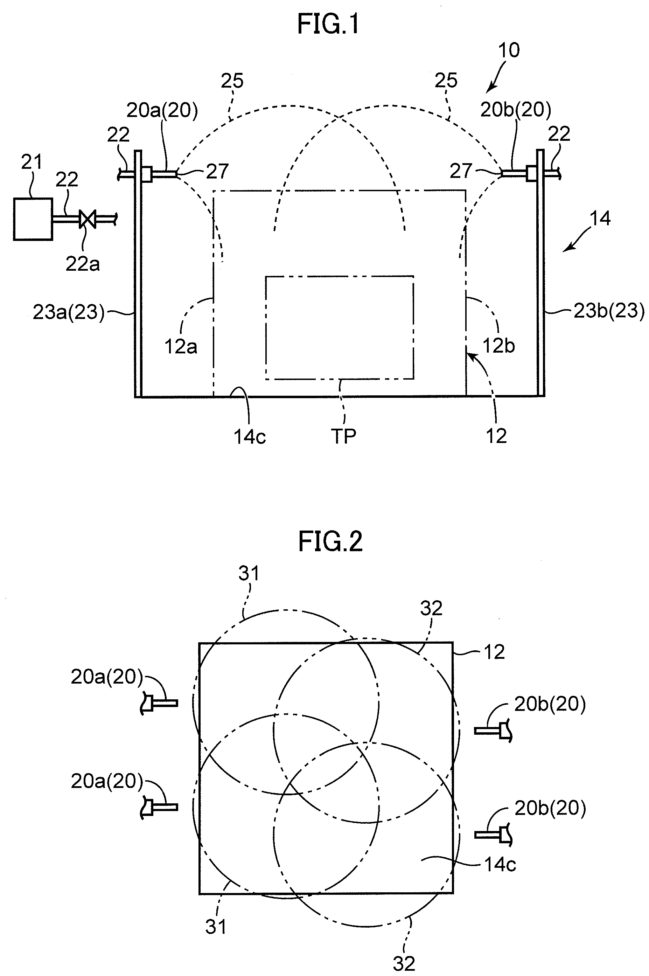

As shown in Figs. 1 and 2, a rainfall test apparatus 10 according to the present embodiment is a test apparatus for evaluating characteristics of a test specimen TP against rainfall by producing rain with rainfall intensity of 10 mm/h or less (or 5 mm/h or less) in a set test area 12. Note that the rainfall test with rainfall intensity of 10 mm/h or less may be performed, for example, with rainfall intensity of 1 mm/h or more, 2 mm/h or more, or 5 mm/h or more.

The test area 12 is an area to be set as an area where desired rainfall intensity is obtained on a floor surface of the test area 12 by spraying water from nozzles 20 described later. The test area 12 may be recognized as an entire test chamber 14 for performing rainfall tests, or as shown in Fig. 1, a predetermined area within the test chamber 14 may be set as the test area 12.

The test area 12 is a cuboid shaped area with a rectangular cross section when viewed from above. The test specimen TP (or at least part of the test specimen TP) is placed within the test area 12. Alternatively, if the test specimen TP is capable of running such as an automobile, the test specimen TP may move to pass through the test area 12 during the rainfall test. Note that the test area 12 does not need to have a cuboid shape, and may have, for example, a cylindrical shape that is a circular shape when viewed from above. That is, a floor surface 14c of the test chamber 14 that constitutes the test area 12 may be either rectangular or circular. The floor surface 14c may have another shape. It is not necessary to set the test area 12 within the test chamber 14. That is, the test area 12 may be set in a building that is not configured as the test chamber 14 or outdoors, and the rainfall test may be performed in the test area 12 inside the building or outdoors.

The rainfall test apparatus 10 includes a plurality of nozzles 20, each configured to spray water droplets 25. Each of the nozzles 20 is connected to a pipe 22 connected to a water supply source 21 and is configured to spray water supplied from the water supply source 21. The pipe 22 is provided with a valve 22a for adjusting the water pressure or flow rate, and by adjusting the opening degree of the valve 22a, the amount and speed of water sprayed from the nozzle 20 change. The valve 22a is adjusted to obtain rain with rainfall intensity of 10 mm/h or less (or 5 mm/h or less).

Note that Fig. 2 shows an example in which four nozzles 20 are disposed, but more nozzles 20 may be disposed or less nozzles 20 may be disposed. In addition, another unillustrated nozzle may be disposed to deliver the water droplets 25 in areas with low rainfall within the test area 12. This unillustrated nozzle may have a water droplet spraying direction different from the nozzles 20. In addition, an unillustrated nozzle may be added to spray water droplets toward the test specimen TP. That is, at least one of the plurality of nozzles only has to be installed to spray the water droplets 25 toward above the test specimen TP such that the water droplets 25 reach the test specimen TP by changing the flow direction of the water droplets downward from the spraying direction.

Two out of the four nozzles 20 (first nozzles 20a) are disposed on one side of the test area 12, while the remaining two (second nozzles 20b) are disposed on the opposite side. That is, each first nozzle 20a is located on one side surface (virtual side surface) 12a or outside of the side surface 12a of the test area 12, and each second nozzle 20b is located on the side surface (virtual side surface) 12b facing the side surface 12a or outside the side surface 12b. In other words, the first nozzle 20a and the second nozzle 20b are disposed on opposite sides of the test specimen TP. Note that the number of the first nozzles 20a and the number of the second nozzles 20b are not limited to two, but a number can be set appropriately according to the size of the test area 12. Each first nozzle 20a may spray the water droplets 25 centered around a direction orthogonal to the side surface 12a when viewed from above. Each second nozzle 20b may spray the water droplets 25 centered around a direction orthogonal to the side surface 12b when viewed from above.

Each first nozzle 20a is fixed to a pillar 23 (first pillar 23a) and is disposed at an appropriate height position according to the size of the test specimen TP. Each second nozzle 20b is fixed to a pillar 23 (second pillar 23b) and is disposed at the same height position as the first nozzle 20a. Note that if the test area 12 is set within the test chamber 14, each first nozzle 20a and each second nozzle 20b may be fixed to side walls of the test chamber 14 instead of being fixed to the pillar 23.

As shown in Fig. 1, the first nozzle 20a and the second nozzle 20b are each installed to spray the water droplets 25 toward above the test specimen TP in the test area 12. Specifically, the first nozzle 20a and the second nozzle 20b are each disposed higher than the upper end of the test specimen TP and are disposed such that a spray port 27 faces horizontally. That is, the nozzles 20 are installed in a posture where the extension line of the spray port 27 passes above the test specimen TP. In Fig. 1, since the spray port 27 faces horizontally, the water droplets 25 are sprayed from the first nozzle 20a and the second nozzle 20b, spreading with the horizontal direction as the center. The water droplets 25 change the flow downward in a parabolic shape by gravity before reaching the test specimen TP, and then reach the test specimen TP. That is, the first nozzle 20a and the second nozzle 20b are installed such that the water droplets 25 sprayed toward above the test specimen TP reach the test specimen TP by changing the flow direction downward from the spraying direction.

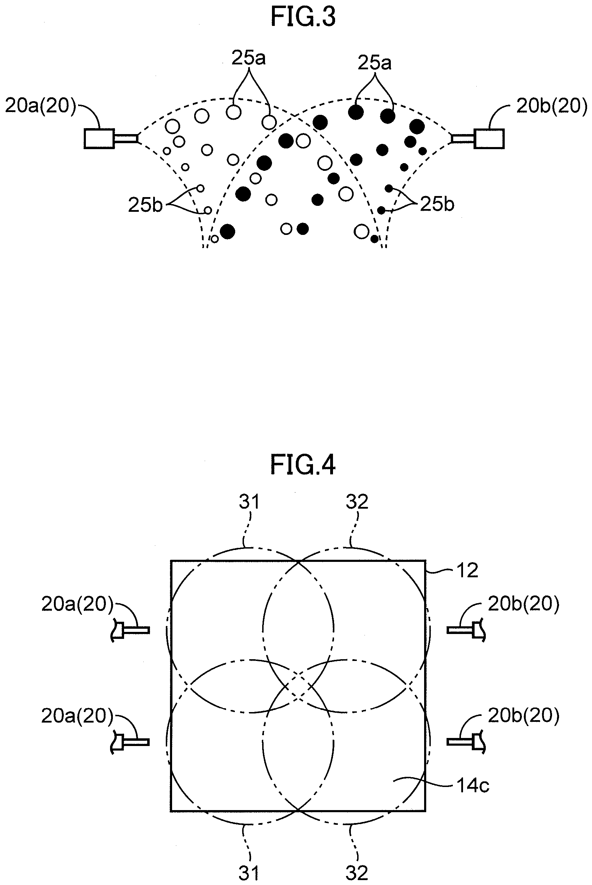

The water droplets 25 are sprayed to spread with the horizontal direction as the center, but there is variation in the size of the water droplets 25. Therefore, the position where the water droplets 25 fall varies depending on the size of the water droplets 25. That is, as shown in Fig. 3, larger water droplets 25a reach farther and smaller water droplets 25b fall closer. By using this property, the positions of the first nozzle 20a and the second nozzle 20b are determined according to the spraying speed such that the water droplets 25 are evenly delivered to the test specimen TP in the test area 12.

That is, the positions of the first nozzle 20a and the second nozzle 20b are set such that the area where the relatively large water droplets 25a out of the water droplets 25 sprayed from the first nozzle 20a fall overlaps with the area where the relatively small water droplets 25b out of the water droplets 25 sprayed from the second nozzle 20b fall. However, as shown in Fig. 2, the first nozzle 20a and the second nozzle 20b do not face each other directly, but are disposed at positions laterally shifted from the facing positions. Therefore, an area 31 where the water droplets 25 from the first nozzle 20a fall and an area 32 where the water droplets 25 from the second nozzle 20b fall are shifted depending on the positional shift between the nozzles 20.

The spacing between first nozzles 20a is set such that a part of the area 31 where the water droplets 25 sprayed from each first nozzle 20a fall overlaps with each other. The spacing between second nozzles 20b is set such that a part of the area 32 where the water droplets 25 sprayed from each second nozzle 20b fall overlaps with each other. This allows the range where the water droplets 25 do not fall to be reduced. Note that Fig. 2 is a diagram for describing the existence of the overlapping area and is merely an illustration. The overlapping area may be larger or smaller than this.

In addition, each first nozzle 20a and each second nozzle 20b are installed such that a part of the water droplets 25 reach a range outside the test area 12, as shown in Fig. 2.

Here, when performing a rainfall test with low rainfall intensity with the rainfall test apparatus 10, there are cases where the supply water pressure to each nozzle 20 is set low in order to reduce the amount of water sprayed from each nozzle 20. When the supply water pressure to each nozzle 20 is reduced, the spray amount from each nozzle 20 decreases, and as a result, the spread of the water droplets 25 sprayed from each nozzle 20 becomes narrower. Then, there is a possibility that it will be difficult to evenly produce rain on the test specimen TP.

To address this possibility, in the present embodiment, the position of the nozzle 20 is set such that some of the water droplets 25 sprayed from the nozzle 20 reach outside the test area 12, in order to reduce the amount of rainfall in the test area 12 without reducing the supply water pressure supplied to each nozzle 20. Note that reducing the supply water pressure and increasing the number of nozzles 20 will ultimately result in increased precipitation, making the test with low rainfall intensity difficult.

Note that in Fig. 2, the first nozzle 20a and the second nozzle 20b are disposed at positions offset laterally (in the up-and-down direction in Fig. 2) from facing positions when viewed from above. However, the arrangement is not limited to this example. For example, as shown in Fig. 4, the first nozzle 20a and the second nozzle 20b may be disposed at positions facing each other when viewed from above. In this case, it is possible to evenly drop the water droplets 25 on the test specimen TP from both sides. Note that even in this case, the area 31 where the water droplets 25 sprayed from the first nozzle 20a fall and the area 32 where the water droplets 25 sprayed from the second nozzle 20b fall may be disposed to partially overlap with each other. The areas 31 onto which the water droplets 25 sprayed from the adjacent first nozzles 20a fall are disposed to partially overlap with each other. However, the arrangement is not limited to this example.

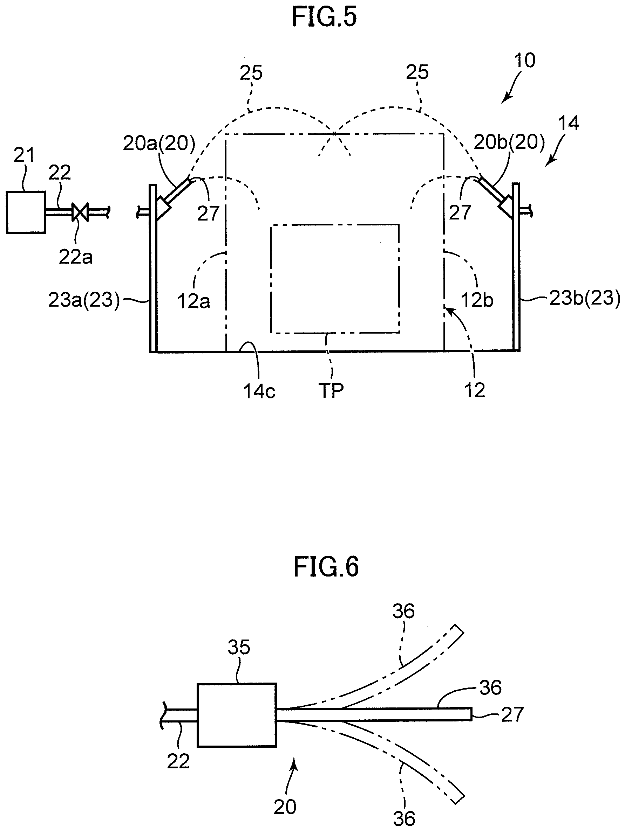

The nozzle 20 is installed with the spray port 27 facing horizontally to spray the water droplets 25 toward above the test specimen TP, as shown in Fig. 1. However, the arrangement is not limited to this example. As shown in Fig. 5, the nozzle 20 may be installed such that the spray port 27 faces obliquely upward. In this case, the water droplets 25 are sprayed to spread with the obliquely upward direction as the center. Note that the nozzle 20 may be installed obliquely downward as long as the water droplets 25 are sprayed toward above the test specimen TP. For example, the nozzle 20 may be installed obliquely downward such that the extension line of the spray port 27 passes above the test specimen TP. In this case, the water droplets 25 are sprayed obliquely downward, and the water droplets 25 are sprayed from the nozzle 20 toward the back side of the test specimen TP. That is, the water droplets 25 are sprayed from the nozzle 20 in a direction that allows the water droplets 25 to reach a position farther than the test specimen TP if the water droplets 25 travel in a straight line. However, after being sprayed, the water droplets 25 gradually change the flow direction downward, resulting in falling on the test specimen TP.

Here, the structure of the first nozzle 20a and the second nozzle 20b will be briefly described. The first nozzle 20a and the second nozzle 20b both have the same structure. As shown in Fig. 6, the nozzle 20 includes a body 35 fixed to the pillar 23 and the like, and a tube 36 connected to the body 35 and having an opening at the tip as the spray port 27. The body 35 is connected to the pipe 22 leading to the water supply source 21, and is configured to allow water sent through the pipe 22 to flow. The body 35 is attached to the pillar 23 and the like such that the spray port 27 of the tube 36 faces a predetermined direction.

The tube 36 allows water that has passed through the body 35 to flow and sprays water from the spray port 27. The tube 36 is thin and flexible to bend easily in an arbitrary direction by the reaction force from the sprayed water. Therefore, when water continues to be sprayed, the tube 36 repeatedly undergoes bending deformation in an arbitrary direction with the body 35 as a support point. As the tube 36 is repeatedly deformed, the sprayed water is divided into the water droplets 25, and the water droplets 25 are sprayed in a scattering manner. Since the tube 36 is configured to bend due to the reaction force of the sprayed water, the amount of deformation changes depending on the supply water pressure. Therefore, as the supply water pressure increases, the spreading range of the water droplets 25 increases, and as the supply water pressure decreases, the spreading range of the water droplets 25 decreases.

Note that the tube 36, which is thin and flexible, can maintain the posture in a natural state where water is not sprayed. Therefore, by determining the orientation of the body 35, the orientation of the spray port 27 can be determined, for example, horizontally.

The nozzle 20 is not limited to the configuration in which the bending deformation of the tube 36 is used to spray water such that the water droplets 25 scatter. For example, the nozzle 20 may be constructed using a hard (for example, metallic) material having the fine spray port 27. In this case, as the water is sprayed from the spray port 27, the sudden expansion causes the water to break up to form the water droplets 25. In this case as well, as the water pressure supplied to the nozzle 20 increases, the spreading range of the water droplets 25 increases. However, for the metallic nozzle 20, since the water droplets 25 are formed by the expansion of the water sprayed from the fine spray port 27, the water needs to be supplied at higher water pressure.

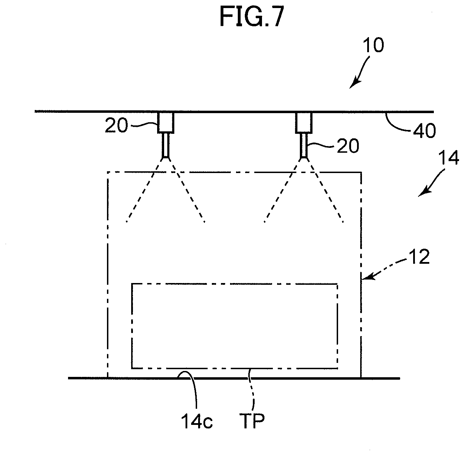

As shown in Fig. 1, the nozzle 20 is fixed to the pillar 23 to spray the water droplets 25 horizontally (spray the water droplets 25 toward above the test specimen TP). Note that when performing rainfall tests with higher rainfall intensity (rainfall intensity higher than 10 mm/h), it is also possible to use the nozzle 20 by changing the position and direction as shown in Fig. 7. That is, the nozzle 20 of Fig. 1 may be removed from the pillar 23 and the like, and the nozzle 20 may be installed to spray the water droplets 25 from top to bottom in the test area 12. In this case, the nozzle 20 is installed such that the spray port 27 faces the test specimen TP instead of facing toward above the test specimen TP, and this is an example of using the nozzle 20 used in the posture shown in Fig. 1 for testing with higher rainfall intensity. In this case, the nozzle 20 may be attached to the ceiling of the test chamber 14 or to a support member 40 positioned above and spanning the test area 12.

As described above, in the rainfall test apparatus 10 of the present embodiment, the plurality of nozzles 20 is installed to spray the water droplets 25 toward above the test specimen TP. The water droplets 25 sprayed from the nozzles 20 reach the test specimen TP by changing the flow direction downward from the spraying direction. Therefore, the travel distance of the water droplets 25 from the nozzles 20 to the test specimen TP is longer than, for example, in the configuration where the nozzles 20 are installed to spray the water droplets 25 toward the test specimen TP as shown in Fig. 7. Furthermore, since the water droplets 25 are sprayed to spread from the nozzles 20, the longer travel distance will cause the water droplets 25 to spread more. Therefore, even if the supply water pressure is reduced to reduce the amount of water to the nozzles 20 such that rain with rainfall intensity of 10 mm/h or less can be obtained, it is possible to suppress the narrowing of the spreading range of the water droplets 25 by increasing the travel distance to the test specimen TP. This makes it possible to reproduce conditions that are closer to actual natural rainfall. Therefore, this configuration allows a smaller amount of the water droplets 25 to fall over a wider range, enabling rainfall tests with low rainfall intensity.

The size of the water droplets 25 sprayed from the nozzles 20 has a distribution. In this case, by installing the nozzles 20 such that the spray port 27 faces horizontally, it is possible to give a distribution according to the size distribution of the water droplets 25 on the floor surface 14c where the water droplets 25 reach. The water droplets 25 sprayed from the nozzles 20 can travel a longer distance than when the spray port 27 is installed to face obliquely downward. Therefore, it is possible to perform a test to produce rain with lower rainfall intensity over a wider range than when the spray port 27 is installed to face obliquely downward. The ceiling of the test chamber 14 can be set lower than when the nozzles 20 are installed such that the spray port 27 sprays the water droplets 25 directly upward, allowing enlargement of the test chamber 14 to be suppressed.

In the present embodiment, the first nozzle 20a and the second nozzle 20b are disposed to spray the water droplets 25 on the test specimen TP from opposite sides. Therefore, uneven rain distribution can be suppressed more than when spraying the water droplets 25 on the test specimen TP from the same side. The first nozzle 20a and the second nozzle 20b are disposed in positions laterally shifted from the positions facing each other. Therefore, it is possible to allow the water droplets 25 from one nozzle 20 to reach the position where the water droplets 25 from the other nozzle 20 are difficult to reach. Among the water droplets 25 sprayed from the first nozzle 20a and the second nozzle 20b, the water droplets 25 with a larger particle size fall far away and the water droplets 25 with a smaller particle size fall close. Therefore, by disposing the first nozzle 20a and the second nozzle 20b as described above, the variation in particle size distribution of the water droplets 25 falling depending on the position on the floor surface 14c can be suppressed.

In the present embodiment, at least a part of the range where the water droplets 25 sprayed from the plurality of nozzles 20 reach is set to overlap with each other. Therefore, for example, the range where relatively large water droplets 25a sprayed from the first nozzle 20a reach and the range where relatively small water droplets 25b sprayed from the second nozzle 20b fall can be set to overlap each other. In this case, there is an effect of resolving uneven precipitation distribution.

### (Second embodiment)

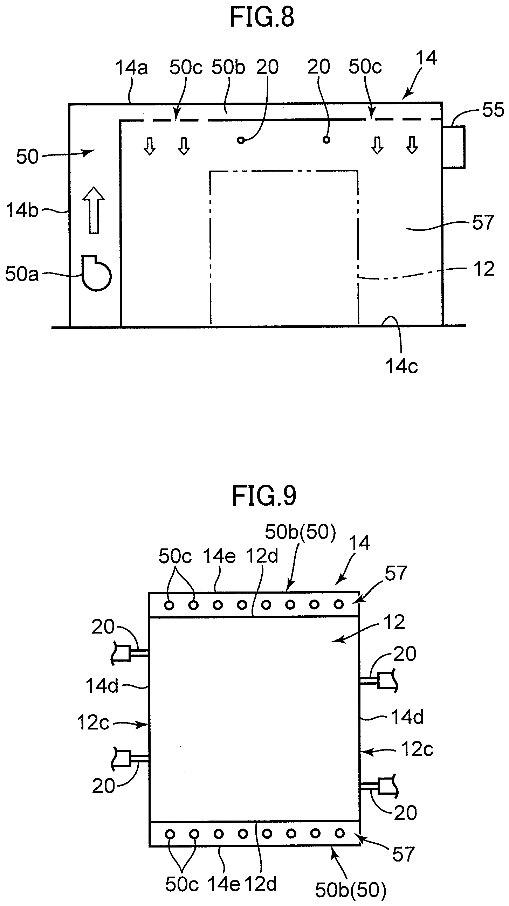

As shown in Fig. 8, in the second embodiment, a blowing unit 50 for supplying air into a test chamber 14 is provided. Note that here, the same components as in the first embodiment are denoted with the same symbols, and detailed descriptions thereof will be omitted.

While an air current is produced in the test chamber 14, it is possible for water droplets 25 sprayed from nozzles 20 to be carried away by the air current in the test chamber 14. Therefore, even if an air current is produced in the test chamber 14, the blowing unit 50 is provided to suppress fluctuations in the rainfall distribution or particle size distribution caused by the water droplets 25 being carried away by the air current.

For example, when air-conditioning the test chamber 14, the conditioned air discharged from an indoor unit 55 of an air conditioner flows to circulate within the test chamber 14. In this case, the water droplets 25 sprayed from the nozzles 20 will be carried away by the conditioned air, and the desired rainfall distribution or particle size distribution may not be obtained. In particular, the water droplets 25b with a small particle size are easily carried away by the air current. Therefore, the blowing unit 50 is configured to generate downflow outside a test area 12 to prevent the water droplets 25 sprayed from the nozzles 20 from being affected by the air current of the conditioned air.

The blowing unit 50 includes a blower 50a for generating the air current and a ventilation path 50b for carrying the air current generated by driving the blower 50a. The ventilation path 50b is disposed along a ceiling 14a of the test chamber 14 above areas including the test area 12 and the outside area (outside area 57). Note that the ventilation path 50b also includes a portion along a side wall 14b of the test chamber 14, and is connected from this portion to an upper portion of the outside area 57.

The ventilation path 50b includes a plate-shaped member disposed to form a space between the ceiling 14a and the side wall 14b of the test chamber 14. That is, the ventilation path 50b is formed by the ceiling 14a and the side wall 14b of the test chamber 14, and the plate-shaped member. However, the ventilation path 50b is not limited to this configuration and may also be constituted by a duct disposed along the ceiling 14a of the test chamber 14. Note that the ventilation path 50b may be provided only above the outside area 57.

The ventilation path 50b is provided with openings 50c that allow air flowing inside the ventilation path 50b to flow out downward. The openings 50c are located above the outside area 57 that surrounds the test area 12, and the downflow is generated around the test area 12 by the air flowing out from the openings 50c. Meanwhile, there is no opening provided above the test area 12. That is, there is no opening provided to allow the air inside the ventilation path 50b to flow out downward directly into the test area 12. Therefore, even if the conditioned air circulates in the test chamber 14, the influence of the flow of conditioned air on the water droplets 25 falling in the test area 12 is suppressed.

Note that a part of the downflow can change the flow direction by a floor surface 14c of the test chamber 14 and flow into the test area 12. However, the openings 50c of the ventilation path 50b cannot be said to be openings that allow the air in the ventilation path 50b to flow out toward the test area 12.

In this way, in the present embodiment, the downflow is generated around the test area 12 by the air flowing out from the openings 50c of the ventilation path 50b. Therefore, even if there is a movement of conditioned air in the outside area 57, the downflow helps to reduce the influence of the flow of conditioned air in the test area 12. This makes it possible to avoid the situation where the water droplets 25 sprayed from the nozzles 20 are carried away by the air flow, and the desired rainfall distribution or particle size distribution cannot be obtained.

Note that the case where the blowing unit 50 for generating the downflow is provided is not limited to the case where the flow of conditioned air is generated in the test chamber 14. Even if there is no flow of conditioned air but there is an air current in the test chamber 14, the same effect can be obtained by providing the blowing unit 50.

The openings 50c of the ventilation path 50b are not limited to the configuration of surrounding the test area 12. For example, as shown in Fig. 9, when the nozzles 20 are provided on each of a pair of opposing side walls 14d of the test chamber 14 that is rectangular when viewed from above, if the side walls 14d define a pair of side surfaces 12c of the rectangular test area 12, the spaces between the other pair of side surfaces 12d of the test area 12 and the other pair of side walls 14e of the test chamber 14 are the outside areas 57. In this case, it is sufficient to provide the openings 50c in each of the outside areas 57 located above and below the test area 12 in Fig. 9.

Descriptions of other configurations, functions, and effects are omitted, but the description of the first embodiment can be cited for the second embodiment.

### (Other embodiments)

Note that it should be appreciated that the embodiments disclosed this time are in all aspects illustrative and not restrictive. The present invention is not limited to the above embodiments, and various changes and modifications can be made without departing from the spirit of the invention. For example, in the above embodiments, the plurality of nozzles 20 is installed to spray the water droplets 25 to the test specimen TP from both opposing sides, but the configuration is not limited to this example. The plurality of nozzles 20 may be installed to spray the water droplets 25 to the test specimen TP from one side.

Here, the above embodiments will be outlined.

(1) The rainfall test apparatus according to the embodiments is a test apparatus for evaluating the characteristics of the test specimen against rainfall by producing rain with rainfall intensity of 10 mm/h or less, and includes nozzles each configured to spray water droplets. Each of the nozzles has a configuration in which a spreading range of the sprayed water droplets changes according to supply water pressure. At least one of the nozzles is installed to spray the water droplets toward above the test specimen such that the water droplets reach the test specimen with a direction of the water droplets changing downward from a spraying direction.

In the rainfall test apparatus, at least one of the nozzles is installed to spray the water droplets toward above the test specimen. The water droplets sprayed from the nozzles reach the test specimen with the flow direction changing downward from the spraying direction. Therefore, the travel distance of the water droplets from the nozzles to the test specimen is longer than in the configuration in which the nozzles are installed to spray the water droplets toward the test specimen. Furthermore, since the water droplets are sprayed to spread from the nozzles, the longer travel distance will cause the water droplets to spread more. Therefore, even if the supply water pressure is reduced to reduce the amount of water to the nozzles such that rain with rainfall intensity of 10 mm/h or less can be obtained, it is possible to suppress the narrowing of the spreading range of the water droplets by increasing the travel distance to the test specimen. This makes it possible to reproduce conditions that are closer to actual rainfall. Therefore, this configuration allows a smaller amount of the water droplets to fall over a wider range, enabling rainfall tests with low rainfall intensity.

(2) The at least one of the nozzles may be installed such that the spray port faces horizontally or obliquely upward.

Since there is distribution in the size of water droplets sprayed from the nozzles, by installing the nozzles such that the spray port faces horizontally or obliquely upward, it is possible to give distribution according to the size distribution of the water droplets on the floor surface where the water droplets reach. In addition, the water droplets sprayed from the nozzles can travel a longer distance than when the spray port is installed to face obliquely downward. Therefore, it is possible to perform a test to produce rain with lower rainfall intensity over a wider range than when the spray port is installed to face obliquely downward. In addition, the ceiling of the test chamber can be set lower than when the nozzles are installed such that the spray port sprays the water droplets directly upward, allowing enlargement of the test chamber to be suppressed.

(3) At least two of the nozzles may be installed to spray the water droplets toward above the test specimen such that the water droplets reach the test specimen with the flow direction of the water droplets changing downward from the spraying direction. In this case, the at least two nozzles may be disposed at positions on opposite sides with respect to the test specimen and shifted laterally from positions facing each other.

This aspect makes it possible to suppress uneven rain distribution more than when spraying the water droplets from the same side of the test specimen. The two nozzles are disposed at positions laterally shifted from the facing positions, allowing the water droplets from one nozzle to reach a position that is difficult to reach from the other nozzle.

(4) At least two of the nozzles may be installed to spray the water droplets toward above the test specimen such that the water droplets reach the test specimen with the flow direction of the water droplets changing downward from the spraying direction. In this case, the at least two nozzles may be disposed on opposite sides of the test specimen to face each other.

This aspect makes it possible to suppress uneven rain distribution more than when spraying the water droplets from the same side of the test specimen. In addition, it is possible to evenly drop the water droplets on the test specimen from both sides.

(5) The at least two nozzles may be disposed such that at least a part of a range where the water droplets reach overlaps with each other.

According to this aspect, for example, the range of water droplets sprayed from one of the two nozzles to reach farther distances may overlap with the range of water droplets sprayed from the other nozzle to reach only shorter distances. The range of water droplets sprayed from the one nozzle to reach only shorter distances may overlap with the range of water droplets sprayed from the other nozzle to reach farther distances. Since relatively large water droplets reach farther and relatively small water droplets only reach closer, if the range of water droplets from the two nozzles overlaps as described above, uneven precipitation distribution is suppressed.

(6) The at least one nozzle may be installed such that a part of the water droplets reaches a range outside the set test area.

According to this aspect, the amount of water droplets falling on the test area decreases more than when all water droplets fall on the test area. This makes it possible to reduce the rainfall intensity (reduce the amount of rainfall) within the test area while maintaining the supply water pressure to the nozzles. Therefore, it is possible to reduce the rainfall intensity while suppressing the narrowing of the spreading range of the water droplets sprayed from the nozzles.

(7) The rainfall test apparatus may include the test chamber and the ventilation path for supplying air into the test chamber. In this case, the ventilation path may have openings that generate downflow outside the test area where the test specimen is placed in the test chamber, while openings that allow the air inside the ventilation path to flow out toward the test area may not be provided.

According to this aspect, the downflow is generated outside the test area by the air flowing out from the openings of the ventilation path. Therefore, even if there is an air movement outside the test area, the downflow can make it difficult for the air inside the test area to be affected by the air movement outside the test area. This makes it possible to avoid the situation where the water droplets sprayed from the nozzles are carried away by the air flow, and desired rainfall distribution or particle size distribution cannot be obtained.

(8) The rainfall test apparatus may include a pipe connected to the nozzles and a valve provided on the pipe to be capable of adjusting the water pressure or flow rate to obtain rain with rainfall intensity of 10 mm/h or less.

(9) The rainfall test method according to the embodiments is a test method for evaluating the characteristics of the test specimen against rainfall by producing rain with rainfall intensity of 10 mm/h or less, and including spraying water droplets from nozzles having a configuration in which the spreading range of the sprayed water droplets changes according to the supply water pressure. At this time, at least one of the nozzles sprays the water droplets toward above the test specimen such that the water droplets reach the test specimen with a flow direction of the water droplets changing downward from the spraying direction.

In the rainfall test method, at least one of the nozzles spray the water droplets toward above the test specimen. The water droplets sprayed from the nozzles reach the test specimen with the flow direction changing downward from the spraying direction. Therefore, the travel distance of the water droplets from the nozzles to reach the test specimen is longer than in the configuration in which the nozzles are installed to spray the water droplets toward the test specimen. Furthermore, since the water droplets are sprayed to spread from the nozzles, the longer travel distance will cause the water droplets to spread more. Therefore, even if the supply water pressure is reduced to reduce the amount of water to the nozzles such that rain with rainfall intensity of 10 mm/h or less can be obtained, it is possible to suppress the narrowing of the spreading range of the water droplets by increasing the travel distance to the test specimen. This makes it possible to reproduce conditions that are closer to actual rainfall. Therefore, this configuration allows a smaller amount of the water droplets to fall over a wider range, enabling rainfall tests with low rainfall intensity.

(10) In the rainfall test method, the method includes adjusting the pressure or flow rate of the water flowing through the pipe toward the nozzles with a valve so as to produce the rain with rainfall intensity of 10 mm/h or less.

As described above, the rainfall test apparatus enables tests with low rainfall intensity of 10 mm/h or less.

This application is based on Japanese Patent application No. 2024-51109 filed in Japan Patent Office on March 27, 2024, the contents of which are hereby incorporated by reference.

## Claims

1. A rainfall test apparatus (10) for evaluating characteristics of a test specimen (TP) against rainfall by producing rain with rainfall intensity of 10 mm/h or less,
the rainfall test apparatus (10) comprising nozzles (20, 20a, 20b) each configured to spray water droplets (25),
wherein each of the nozzles (20, 20a, 20b) has a configuration in which a spreading range of the sprayed water droplets (25) changes according to supply water pressure, and
at least one of the nozzles (20, 20a, 20b) is installed to spray the water droplets (25) toward above the test specimen (TP) such that the water droplets (25) reach the test specimen (TP) with a flow direction of the water droplets (25) changing downward from a spraying direction.

2. The rainfall test apparatus (10) according to claim 1, wherein the at least one of the nozzles (20, 20a, 20b) is installed such that a spray port (27) faces horizontally or obliquely upward.

3. The rainfall test apparatus (10) according to claim 1 or 2, wherein
at least two nozzles (20, 20a, 20b) of the nozzles (20, 20a, 20b) are installed to spray the water droplets (25) toward above the test specimen (TP) such that the water droplets (25) reach the test specimen (TP) with the flow direction of the water droplets (25) changing downward from the spraying direction, and
the at least two nozzles (20, 20a, 20b) are disposed at positions on opposite sides with respect to the test specimen (TP) and shifted laterally from positions facing each other.

4. The rainfall test apparatus (10) according to claim 1 or 2, wherein
at least two nozzles (20, 20a, 20b) of the nozzles (20, 20a, 20b) are installed to spray the water droplets (25) toward above the test specimen (TP) such that the water droplets (25) reach the test specimen (TP) with the flow direction of the water droplets (25) changing downward from the spraying direction, and
the at least two nozzles (20, 20a, 20b) are disposed on opposite sides with respect to the test specimen (TP) to face each other.

5. The rainfall test apparatus (10) according to claim 3 or 4, wherein the at least two nozzles (20, 20a, 20b) are disposed such that at least a part of a range where the water droplets (25) reach overlaps with each other.

6. The rainfall test apparatus (10) according to any one of claims 1 to 5, wherein the at least one nozzle (20, 20a, 20b) is installed such that a part of the water droplets (25) reaches a range outside a set test area (12).

7. The rainfall test apparatus (10) according to any one of claims 1 to 6, further comprising: a test chamber (14); and a ventilation path (50b) for supplying air into the test chamber (14),
wherein the ventilation path (50b) includes an opening (50c) that generates downflow outside the test area (12) where the test specimen (TP) is placed inside the test chamber (14), while no opening that allows the air inside the ventilation path (50b) to flow out toward the test area (12) is provided.

8. The rainfall test apparatus (10) according to any one of claims 1 to 7, further comprising:
a pipe (22) connected to the nozzles (20, 20a, 20b); and
a valve (22a) provided on the pipe (22) to be capable of adjusting pressure or a flow rate of water to obtain the rain with the rainfall intensity of 10 mm/h or less.

9. A rainfall test method for evaluating characteristics of a test specimen (TP) against rainfall by producing rain with rainfall intensity of 10 mm/h or less, the rainfall test method comprising
spraying water droplets (25) from nozzles (20, 20a, 20b) having a configuration in which a spreading range of the sprayed water droplets (25) changes according to supply water pressure,
in the spraying water droplets (25), at least one of the nozzles (20, 20a, 20b) sprays the water droplets (25) toward above the test specimen (TP) such that the water droplets (25) reach the test specimen (TP) with a flow direction of the water droplets (25) changing downward from a spraying direction.

10. The rainfall test method according to claim 9, further comprising adjusting pressure or a flow rate of water flowing through a pipe (22) toward the nozzles (20, 20a, 20b) with a valve (22a) so as to produce the rain with the rainfall intensity of 10 mm/h or less.
